# EUROPEAN PATENT SPECIFICATION

(11) **EP 2 627 303 B1**
(45) Date of publication and mention of the grant of the patent: **31.08.2016**
(21) Application number: 11773630.6
(22) Date of filing: 11.10.2011
(51) Int. Cl.: A61K 8/11, A61K 8/34, A61K 8/73, A61K 8/81, A61K 8/86, A61K 8/87, A61K 8/92, A61Q 9/02, B26B 21/44

(54) **A SKIN ENGAGING MEMBER COMPRISING ENCAPSULATED ACTIVES**
HAUTKONTAKTELEMENT ENTHALTEND VERKAPSELTE WIRKSTOFFE
ÉLÉMENT ENTRANT EN CONTACT AVEC LA PEAU ET COMPRENANT DES SUBSTANCES ACTIVES ENCAPSULÉES

(30) Priority: 11.10.2010 US 391891 P
(43) Date of publication of application: 21.08.2013
(73) Proprietor: The Gillette Company, Boston, MA 02127 (US)
(72) Inventor: WANG, Xiandong, Acton, Massachusetts 01720 (US); KWIECIEN, Michael, Joseph, Scituate, Massachusetts 02066 (US)
(74) Representative: Kohol, Sonia
(86) International application number: PCT/US2011/055740
(87) International publication number: WO 2012/051166

(56) References cited:
- US-A- 5 653 971
- US-B2- 7 115 282

## Description

### BACKGROUND OF THE INVENTION

The use of shaving aids on razor blades to provide lubrication benefits during the shave is known. *See e.g*., U.S. Patents 7,121,754; 6,298,558; 5,711,076; 5,134,775; and U.S. Patent Publ. Nos. 2009/0223057, 2006/0225285.

The addition of various actives into shaving aids has also been attempted. For example, it has been described that cooling agents and/or essential oils can be included in the shaving aid to deliver a fresh and cool feel after contact. It has been reported, however, that a substantial amount of the essential oil can be lost due to volatilization prior to use. *See* U.S. Patent No. 5,095,619. U.S. Patent No. 5,713,131 attempts to fix this potential problem by introducing non-volatile cooling agents into the shave aid, such as non-volatile menthol analogs. Examples of other shave aids containing menthol and other actives are disclosed in U.S. Patents 5,095,619, 6,298,558, 6,944,952, and 6,295,733. The addition of cyclodextrin inclusion complexes and displacing agents have also been described in U.S. Patent Nos. 5,653,971, and, 5,713,131.

The processing temperatures to make a shaving aid via extrusion are typically in the range of 160°C. Many of the known encapsulation technologies cannot survive processing temperatures around 160°C and high shear forces during the extrusion process. Some technologies may partially survive the process, but fail to deliver the desired amount and / or effectiveness of the cooling agent during wet shaving. Many other encapsulation technologies, however, may be more resilient to temperature and processing conditions but may not be safe for use in consumer goods (i.e., melamine formaldehyde and/or certain gelatin capsules).

As such, despite the numerous attempts to incorporate cooling agents into shaving aids, there remains a need for new shaving aid formulations which are less susceptible to damage to the cooling agents during the making process yet are still suitable for use in a topological skin care treatment.

### SUMMARY OF THE INVENTION

This invention relates to a skin engaging member, attached to a razor cartridge for use with a hair removal device, such as a razor or depilatory and scraping tool, said skin engaging member comprising a matrix comprises at least one of: a water soluble polymer, an emollient, and a mixture thereof; an encapsulated active comprising an encapsulated active comprising at least one nano-particle encapsulated in a micro-particle, wherein said nano-particle comprises a shell comprising a hydrophobic material, and wherein said micro-particle comprises a shell comprising a water sensitive material; and wherein at least one of said nano-particle and said micro-particle comprises a skin care active, wherein said encapsulated actives is in said matrix material.

### BRIEF DESCRIPTION OF THE DRAWINGS

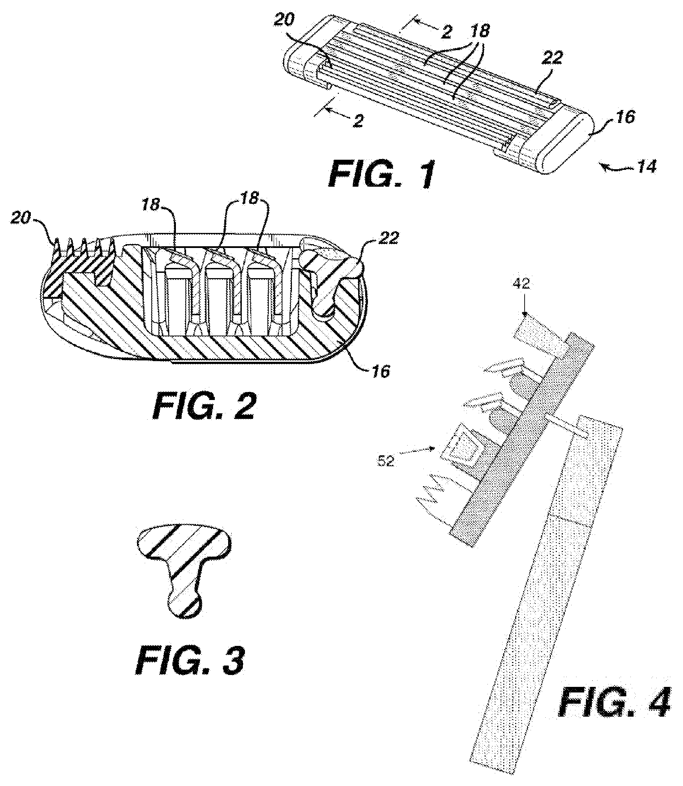

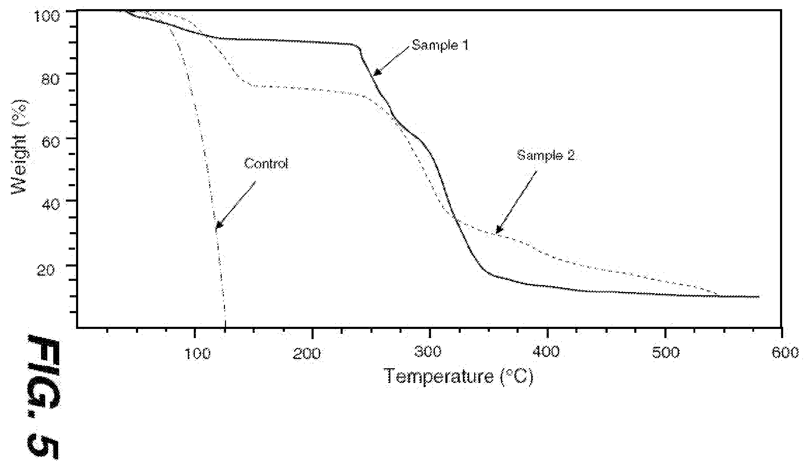

Fig. 1 is a perspective view of a razor cartridge which includes a skin engaging member of the present invention. Fig. 2 is a sectional view taken along line 2-2 of Fig. 1. Fig. 3 is a side elevation view of second type of skin engaging member of the present invention. Fig. 4 is a side view of another razor in accordance with the present invention. Fig. 5 is a chart showing the thermogravimetric analysis of an encapsulated active of the present invention compared to another encapsulated active and a non-encapsulated active.

### DETAILED DESCRIPTION OF THE INVENTION

The skin engaging member of the present invention comprises at least one encapsulated active, and optional other skin engaging members. The encapsulated active can be a coating on the exterior of the skin engaging member, can be provided as a discrete layer or layers in the skin engaging member, or can be mixed into the composite of the skin engaging member. The skin engaging member can be used on a hair removal device, including but not limited to blades and razors and provides a cooling benefit on skin during and/or after contact with skin.

Prior to addition into the skin engaging member, the encapsulated actives of the present invention can be a free-flowing powder formed of solid hydrophobic nano-particles comprising at least one cooling agent, which is encapsulated in a moisture sensitive micro-particles that can also contain at least one cooling agent. The active ingredients encapsulated in the nano-particles can be the same or different from those encapsulated in the micro-particle. Those of skill in the art will understand that the nano-particles and micro-particles referred to herein include capsules but can also include non fully encapsulated particles.

### I. Encapsulated Actives

The skin engaging member of the present invention comprises at least one encapsulated active. In one embodiment, the level of said at least one encapsulated active is from about 0.01 % to about 50 % by weight of said skin engaging member, alternatively from about 10 % to about 45 %, alternatively from about 15 % to about 35 %. In one embodiment, the skin engaging member comprises more than one encapsulated active, meaning that either the cooling agent(s) contained with the encapsulated actives and/ or the materials used to make the nano-particle and/or micro-particles are different.

### a. Nano-Particles and Micro-Particles

In one embodiment, the nano-particles and micro-particles are the nano-spheres and/or micro-spheres generally described in U.S. Patent No. 7,115,282. The term "particles" is intended to describe solid, substantially spherical particulates. It will be appreciated that other shapes can be formed in accordance with the teachings of the present invention. The nano-particles of the present invention are hydrophobic in nature. The nano-particles have an average diameter in the range from about 0.01 micron to about 10 microns, or from about 0.05 microns to about 5 microns, or from about 0.1 microns to about 2 microns. This linear dimension for any individual particle represents the length of the longest straight line joining two points on the surface of the particle.

In one embodiment, a portion of the nano-particles are encapsulated into one or more water-sensitive micro-particles. In one embodiment, the majority of the nano-particles present in the skin engaging member are encapsulated into said water-sensitive micro-particles. The micro-particles have an average particle size of from about 2.0 microns to about 100 microns, or from 20 microns to about 100 microns or from 20 microns to about 100 microns.

### i. Capsule Materials for Forming the Nano-Particles

Suitable capsule materials for forming the nano-particles of the present invention are inert nontoxic hydrophobic materials with a melting point range between about 30 °C and about 90 °C. Examples of hydrophobic materials include natural, regenerated, or synthetic waxes including animal waxes such as beeswax, lanolin and shellac wax, vegetable waxes such as carnauba, candelilla, sugar cane, rice bran, and bayberry wax, mineral waxes such as petroleum waxes including paraffin and microcrystalline wax, and mixtures thereof. Other hydrophobic materials which can be used in the present invention include wax and silicon copolymers, such as candelilla wax and silicone copolymer, ozokrite wax and silicon copolymers, beeswax and silicon copolymers.

Other hydrophobic compounds which can be used in the present invention include: fatty acid esters such as ethyl stearate, isopropyl myristate, and isopropyl palmitate; high mol.wt. fatty alcohols such as cetostearyl alcohol, cetyl alcohol, stearyl alcohol, and oleyl alcohol, solid hydrogenated castor and vegetable oils, hard paraffins, hard fats, and mixtures thereof. Other hydrophobic compounds which can be used, include triglycerides, preferably of at least food grade purity, which can be produced by synthesis or by isolation from natural sources. Natural sources can include animal fat or vegetable oil, such as soy oil, as a source of long chain triglycerides (LCT). Other triglycerides suitable for use in the present invention are composed of a majority of medium length fatty acids (C10-18), denoted medium chain triglycerides (MCT). The fatty acid moieties of such triglycerides can be unsaturated or polyunsaturated and mixtures of triglycerides having various fatty acid material. The nano-particle capsule material can comprise a single hydrophobic material or a mixture of a plurality of materials. Other suitable hydrophobic materials that are known to those skilled in the art are described in "Industrial Waxes," Vol. I and II, by Bennett F.A.I.C., published by Chemical Publishing Company Inc., 1975 and Martindale, "The Extra Pharmacopoeia", The Pharmaceutical Press, 28th Edition pp.1063-1072, 1982.

Other hydrophobic compounds which can be used in the present invention include synthetic polymers, such as alkylated polyvinylpyrrolidines, the Ganex® copolymer series, and ProLipid® 151, commercially available from the ISP Company. Examples of other suitable hydrophobic polymers and copolymer for use as the capsule material include polyethylene homopolymers A-C® 1702; A-C® 617, A-C® 617A, and A-C® 15, commercially available from Allied Signal Inc.; PERFORMALENE™ PL commercially available from New Phase Technologies; ETHYLENE-ACRYLIC ACID COPOLYMERS A-C® 540, A-C® 540A, and A-C® 580 commercially available from Allied Signal Inc.; polyamides having a mol.wt. in the range of from about 6,000 up to about 12,000, for example, MACROMELT™ 6030 manufactured by the Henkel Ag. of Dusseldorf, Germany; VERSALON™ 1135 polyamide polymer available from General Mills, Inc

The nano-particles of the present invention can have a melting point in the range from about 30 °C to about 90 °C, preferably from about 40 °C to about 90 °C The melting point of the particles is usually a function of the carrier matrix employed. Accordingly, preferred matrix materials have a melting point in the range of about 50 °C to about 80 °C, preferably from about 60 °C to about 70 °C. Considerations in the selection of the matrix material include good barrier properties to the active agents and the fragrance ingredients, low toxicity and irritancy, stability, and high loading capacity for the active agents of interest.

### ii. Capsule Materials for Forming the Micro-Particles

Water-sensitive materials for forming the micro-particles of the present invention comprise water soluble and water dispersible synthetic polymers and copolymers, starch derivatives, polysaccharides, hydrocolloids, natural gums, proteins, and mixtures thereof.

Examples of synthetic water sensitive polymers which are useful for the invention include polyvinyl pyrrolidone, water soluble celluloses, polyvinyl alcohol, ethylene maleic anhydride copolymer, methylvinyl ether maleic anhydride copolymer, acrylic acid copolymers, anionic polymers of methacrylic acid and methacrylate, cationic polymers with dimethyl-aminoethyl ammonium functional groups, polyethylene oxides, water soluble polyamide or polyester.

Examples of water soluble hydroxyalkyl and carboxyalkyl celluloses include hydroxyethyl and carboxymethyl cellulose, hydroxyethyl and carboxyethyl cellulose, hydroxymethyl and carboxymethyl cellulose, hydroxypropyl carboxymethyl cellulose, hydroxypropyl methyl carboxyethyl cellulose, hydroxypropyl carboxypropyl cellulose, hydroxybutyl carboxymethyl cellulose, and the like. Also useful are alkali metal salts of these carboxyalkyl celluloses, particularly and preferably the sodium and potassium derivatives.

Polyvinyl alcohol useful in the practice of the invention is partially and fully hydrolyzed polyvinyl acetate, termed "polyvinyl alcohol" with polyvinyl acetate as hydrolyzed to an extent, also termed degree of hydrolysis, of from about 75% up to about 99%. Such materials are prepared by means of any of Examples I-XIV of U.S. Pat. No. 5,051,222 issued on Sep. 24, 1991.

A polyvinyl alcohol useful for practice of the present invention is Mowiol® 3-83, having a mol.wt. of about 14,000 Da and degree of hydrolysis of about 83%, Mowiol® 3-98 and a fully hydrolyzed (98%) polyvinyl alcohol having a mol.wt. of 16,000 Da commercially available from Gehring-Montgomery, Inc. of Warminister Pa. Other suitable polyvinyl alcohols are: AIRVOL® 205, having a mol.wt. of about 15,000-27,000 Da and degree of hydrolysis of about 88%, and VINEX® 1025, having mol.wt. of 15,000-27,000 Da degree of hydrolysis of about 99% and commercially available from Air Products & Chemicals, Inc. of Allentown, Pa.; ELVANOL® 51-05, having a mol.wt. of about 22,000-26,000 Da and degree of hydrolysis of about 89% and commercially available from the Du Pont Company, Polymer Products Department, Wilmington, Del.; ALCOTEX® 78 having a degree of hydrolysis of about 76% to about 79%, ALCOTEX® F88/4 having a degree of hydrolysis of about 86% to about 88% and commercially available from the Harlow Chemical Co. Ltd. of Templefields, Harlow, Essex, England CM20 2BH; and GOHSENOL® GL-03 and GOHSENOL® KA-20 commercially available from Nippon Gohsei K.K., The Nippon Synthetic Chemical Industry Co., Ltd., of No. 9-6, Nozaki Cho, Kita-Ku, Osaka, 530 Japan.

Suitable polysaccharides are polysaccharides of the non-sweet, coloidally-soluble types, such as natural gums, for example, gum arabic, starch derivates, dextrinized and hydrolyzed starches, and the like. A suitable polysaccharide is a water dispersible, modified starch commercially available as Capule®, N-Lok®, Hi-Cap™ 100 or Hi-Cap™ 200 commercially available from the National Starch and Chemical Company of Bridgewater, N.J.; Pure-Cote™, commercially available from the Grain Processing Corporation of Muscatine, Iowa. In the preferred embodiment the natural gum is a gum arabic, commercially available from TIC Gums Inc. Belcamp, Midland. Suitable hydrocolloids are xanthan, maltodextrin, galactomanan or tragacanth, preferably maltodextrins such as Maltrin™ M100, and Maltrin™ M150, commercially available from the Grain Processing Corporation of Muscatine, Iowa.

### b. Method of making the nano and micro-particles

### i. Nano-Particles

The encapsulated actives in the nano-particles of the present invention can be prepared by the steps of (1) heating hydrophobic materials to a temperature above the melting point to form a melt, (2) dissolving or dispersing the said active ingredients in the melt, (4) emulsifying the melt in the aqueous phase; and (5) cooling the dispersion to ambient temper to form a fine suspension. The active ingredients can be incorporated into the hydrophobic solid nano-particles. Preferably, about 1% to about 80% of and more preferably about 1% to about 60% by weight of the active ingredients are used in forming the nano-particles.

### ii. Micro-particles

The encapsulated actives of the present invention can be prepared by the steps of (a) incorporating a first active into the hydrophobic interior of the nano-particles, (b) forming an aqueous mixture comprising a second active, the nano-particles, and a water sensitive material, and (c) spray drying the mixture of the present invention to form a dry powder composition. Accordingly, the nano-particles can be encapsulated into the micro-particle structure. The first and second actives can be the same or different, or there could just be actives in the nano particles, or the micro particles.

A process for producing the multi component controlled release system can include the following steps:
(i) heating hydrophobic materials to a temperature above the melting point of the materials to form a melt;
(ii) dissolving or dispersing the first said fragrance or flavor into the melt;
(iii) dissolving or dispersing the first said active ingredients into the melt;
(iv) dissolving or dispersing a second active ingredients, and moisture sensitive materials, such as, starch derivatives, natural gums, polyvinyl alcohol, proteins, hydrocolloids, or mixture of thereof, in the aqueous phase;
(v) heating the composition to above the melting temperature of the hydrophobic materials;
(vi) mixing the hot melt with the aqueous phase to form a dispersion;
(vii) high shear homogenization of the dispersion at a temperature above the melting temperature until a homogeneous fine dispersion is obtained having a particle size of from about 1 micron to about 2 microns;
(viii) cooling the dispersion to ambient temperature; and
(ix) spray drying the emulsified mixed suspension to form a dry powder composition.

This dry powder composition can then be used in the method of making the skin engaging member of the present invention. Additional details on the method of making the nano-particles and micro-particles are disclosed in U.S. Patent No. 7,115,282 (in reference to nano-spheres and micro-spheres).

### c. Skin Care Active Ingredients in the Encapsulated Actives

Various skin care actives ("actives") which are commonly used for topical application can be present in the nano-particles and/or micro-particles. In one embodiment, the encapsulated active contains one or more active ingredient, including but not limited to a cooling agent. Non-limiting examples of suitable cooling agents include: L-menthol; p-methane-3,8-diol; Isopulegol; Menthoxypropane-1,2,-diol; Curcumin; Menthyl Lactate (such as Frescolat ML by Symrise); Gingerol; Icilin; Tea Tree Oil; Methyl Salicylate; Camphor; Peppermint Oil; N-Ethyl-p-menthane-3-carboxanide; Ethyl 3-(p-menthane-3-carboxamido)acetate; 2-Isopropyl-N,2,3-trimethylbutyramide; Menthone glycerol ketal, Menthone Glyerine Acetal; Coolact 10; and mixtures thereof. These and other cooling agents are known and described in various publications, such as U.S. Patent No. 2008/0300314A1, U.S. Patent No. 5,451,404 and 7,482,373. In yet another embodiment, the cooling agent comprises one or more of the cooling agents previously described for use in various shave aids. *See e.g*., U.S. Patent Nos. 5,095,619; 5,713,131; 5,095,619; 5,653,971; 6,298,558; 6,944,952; and 6,295,733.

Other actives suitable for cosmetic and dermatological use can be used herein. Non-limiting examples of suitable actives include one or more of: Bis-abolol and ginger extract, a surfactant derived from olive oil such as Olivem 450® and Olivem 460®, Lauryl p-Cresol Ketoxime, 4-(1-Phenylethyl)1,3-benzenediol, Lupin (Lupinus albus) oil & wheat (Triticum vulgare) germ oil unsaponifiables, Hydrolyzed lupin protein, Extract of L-lysine and L-arginine peptides, Oil soluble vitamin C, Evodia rutaecarpa fruit extract, Zinc pidolate and zinc PCA, Alpha-linoleic acid, p-thymol, and combinations thereof; at least one additional skin and/or hair care active selected from the group consisting of sugar amines, vitamin B₃, retinoids, hydroquinone, peptides, farnesol, phytosterol, dialkanoyl hydroxyproline, hexamidine, salicylic acid, N-acyl amino acid compounds, sunscreen actives, water soluble vitamins, oil soluble vitamins, hesperedin, mustard seed extract, glycyrrhizic acid, glycyrrhetinic acid, carnosine, Butylated Hydroxytoluene (BHT) and Butylated Hydroxyanisole (BHA), menthyl anthranilate, cetyl pyridinium chloride, tetrahydrocurmin, vanillin or its derivatives, ergothioneine, melanostatine, sterol esters, idebenone, dehydroacetic acid, Licohalcone A, creatine, creatinine, feverfew extract, yeast extract (e.g., Pitera®), beta glucans, alpha glucans, diethylhexyl syringylidene malonate, erythritol, p-cymen-7-ol, benzyl phenylacetate, 4-(4-methoxyphenyl)butan-2-one, ethoxyquin, tannic acid, gallic acid, octadecenedioic acid, p-cymen-5-ol, methyl sulfonyl methane, an avenathramide compound, fatty acids (especially polyunsaturated fatty acids), anti-fungal agents, thiol compounds (e.g., N-acetyl cysteine, glutathione, thioglycolate), other vitamins (vitamin B 12), beta-carotene, ubiquinone, amino acids, their salts, their derivatives, their precursors, and/or combinations thereof; and a dermatologically acceptable carrier. These and other potentially suitable actives are described in greater detail in U.S. Patent Publication No. 2008/0069784.

Additional actives that can be used include those commercially available under the following tradenames: Signaline S, Jojoba Oil, Ceramidone, Net DG, Pal-GHK (Paltenex), Rhodysterol, Vital ET, and combinations thereof.

In another embodiment, the active can be a methyl naphthalenyl ketone. The methyl naphthalenyl ketone can be a 1-(1,2,3,4,5,6,7,8-octahydro-2,3,8,8-tetramethyl-2naphthalenyl)-ethan-1-one molecule or an isomer or derivative thereof. Commercially available as Iso-E-Super from IFF of New York. Other sensates can also be used, including those which have ability to up-regulate the TRPM8 receptor, which has been described as the cool menthol receptor. Non-limiting examples of suiltable TRPM8 regulators include: p-methane-3,8-diol; Isopulegol; Menthoxypropane-1,2,-diol; Curcumin; Menthyl Lactate; Gingerol; Icilin; Menthol; Tea Tree Oil; Methyl Salicylate; Camphor; Peppermint Oil; N-Ethyl-p-menthane-3-carboxamide; Ethyl 3-(p-menthane-3-carboxamido)acetate; 2-Isopropyl-N,2,3-trimethylbutyramide; Menthone glycerol ketal, and mixtures thereof.

In one embodiment the level of active or actives in the encapsulated active ranges from about 20 to about 90%, preferably from about 30 % to about 75% by weight of the nano-particles. In one embodiment the level of the active or actives in the encapsulated active ranges from about 10% to about 60%, or from about 30% to about 50% by weight of the micro-particles.

In one embodiment, encapsulated active comprises more than one cooling agent, for example L-menthol + Menthyl lactate (Frescolat ML); L-menthol + Menthone Glycerine Acetal (Frescolat MGA); or L-menthol + Coolact 10. In yet another embodiment, the encapsulated active comprises at least one cooling agent and a fragrance, a mineral oil, or a combination thereof. In another embodiment, the cooling agent comprises a mixture of menthol and menthyl lactate, such as described in WO 2007115593 (commercially available as Fresocolat Plus), or the eutectic mixture of menthol and menthyl lactate in a ratio of weight in the range of 1:4 to 4:1, as described in U.S. 6,897,195.

Without intending to be bound by theory, it is believed that the skin engaging member comprising the encapsulated actives of the present invention are preferred over other known shaving aids comprising neat cooling agents or encapsulated cooling agents because the specific type of encapsulation involved allows more of the cooling agent to survive the manufacturing process. As defined herein, "neat" refers to actives which are not encapsulated in either the nano-particle or the micro-particle, but are dispersed within the skin engaging member.

In one embodiment, the nano-capsule is made of a hydrophobic low melting material providing a lower shear strength material which can rupture upon elevated temperature or/and wearing against rough surface. As defined herein, low melting means a melting point below 30 °C, one example of a low melting material is shea butter. As explained above, the micro-encapsulating material is a water soluble and/or water dispersible material. The ingredient within such micro-particles can be triggered to release by moisture, warm or cold water or aqueous liquid. A list of compositions is given in Table 1, below in the Examples section.

In one embodiment, only one active is in the capsule. In another embodiment, there are two or more types of active ingredients are encapsulated. These two ingredients may function similarly or differently in terms of providing skin or shaving benefits. In one embodiment the two or more actives are selected to be chemically or biologically compatible, and may thus be combined together for encapsulation process (meaning the mixture can exist in the same nano- or micro-capsule. If the two or more actives are not compatible, they may be encapsulated in separate nano- and/or micro-particles, or the first active can be in the nano-particles and the second active can be external to the nano-particle but within the micro-particles. In anther embodiment, the encapsulated active includes a plurality of nano-capsules with the first active, the second active, or a combination thereof. External to the nano-capsule can exist the first active, second active, or a mixture thereof. It is believed that such specific encapsulated actives can deliver the desired amount of one or more incompatible actives during the shaving or hair removal process.

It is believed that many factors will affect the release of active ingredient under specific application. Those factors may include wall thickness, distribution of active ingredient between the two different type particles, and the integration of nano-particles with micro-particles capsule materials, as well as use conditions.

The active ingredient can also be one or more skin care actives suitable for topical use. The CTFA Cosmetic Ingredient Handbook, Second Edition (1992) describes a wide variety of nonlimiting cosmetic and pharmaceutical ingredients commonly used in the skin care industry, which are suitable for use in the compositions of the present invention. Examples of these ingredient classes include: abrasives, absorbents, aesthetic components such as fragrances, pigments, colorings/colorants, essential oils, skin sensates, astringents, etc. (e.g., clove oil, camphor, eucalyptus oil, eugenol, witch hazel distillate), anti-acne agents, anti-caking agents, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, fatty alcohols and fatty acids, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents, skin-conditioning agents, skin soothing and/or healing agents and derivatives, skin treating agents, thickeners, and vitamins and derivatives thereof. Additional non-limiting examples of additional suitable skin treatment actives are included in U.S. 2003/0082219 in Section I (i.e. hexamidine, zinc oxide, and niacinamide); U.S. 5,665,339 at Section D (i.e. coolants, skin conditioning agents, sunscreens and pigments, and medicaments); and US 2005/0019356 (i.e. desquamation actives, anti-acne actives, chelators, flavonoids, and antimicrobial and antifungal actives). It should be noted, however, that many materials may provide more than one benefit, or operate via more than one mode of action. Therefore, classifications herein are made for the sake of convenience and are not intended to limit the active to that particular application or applications listed.

### II. Matrix Material

The skin engaging comprises the encapsulated active in or on a matrix material. The matrix material can be in the form of a solid polymeric matrix or an emollient.

### a. Solid Polymeric Matrix

In one embodiment, the matrix comprises a water soluble polymer comprising at least one of a polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and a mixtures thereof. In one embodiment, said water soluble polymer is selected from the group consisting of polyethylene oxide, polyethylene glycol, and a mixture thereof.

In one embodiment, the skin engaging member comprises any other ingredients commonly found in commercially available shaving aids, such as those used on razor cartridges by Gillette, Schick or BIC. Non-limiting examples of such shaving aids include those disclosed in U.S. 6301785, 6442839, 6298558, 6302785, 2008/060201, and 2009/0223057. In one embodiment, the skin engaging member further comprises a shaving aid ingredient selected from the group consisting of polyethylene oxide, polyvinyl pyrrolidone, polyacrylamide, hydroxypropyl cellulose, polyvinyl imidazoline, polyethylene glycol, poly vinyl alcohol, polyhydroxyethylmethacrylate, silicone copolymers, sucrose stearate, vitamin E, soaps, surfactants, panthenol, aloe, plasticizers, such as polyethylene glycol; beard softeners; additional lubricants, such as silicone oil, Teflon® polytetrafluoroethylene powders (manufactured by DuPont), and waxes; essential oils such as menthol, camphor, eugenol, eucalyptol, safrol and methyl salicylate; tackifiers such as Hercules Regalrez 1094 and 1126; non-volatile cooling agents, inclusion complexes of skin-soothing agents with cyclodextrins; fragrances; antipruritic/counterirritant materials; antimicrobial/keratolytic materials such as Resorcinol; antiinflammatory agents such as Candilla wax and glycyrrhetinic acid; astringents such as zinc sulfate; surfactants such as pluronic and iconol materials; compatibilizers such as styrene-b-EO copolymers; mineral oil, polycaprolactone (PCL), and combinations thereof.

The water-soluble polymer will preferably comprise at least 50%, more preferably at least 60%, by weight of the skin engaging member, up to about 99%, or up to about 90% of the matrix. The more preferred water soluble polymers are the polyethylene oxides generally known as POLYOX (available from Union Carbide Corporation) or ALKOX (available from Meisei Chemical Works, Kyoto, Japan). These polyethylene oxides will preferably have mol.wt.s of about 100,000 to 6 million, most preferably about 300,000 to 5 million. The most preferred polyethylene oxide comprises a blend of about 40 to 80% of polyethylene oxide having an average mol.wt. of about 5 million (e.g. POLYOX COAGULANT) and about 60 to 20% of polyethylene oxide having an average mol.wt. of about 300,000 (e.g. POLYOX WSR-N-750). The polyethylene oxide blend may also advantageously contain up to about 10% by weight of a low mol.wt. (i.e. MW<10,000) polyethylene glycol such as PEG-100.

In one embodiment, the matrix further comprises from about 0.5% to about 50%, preferably from about 1% to about 20%, polycaprolactone (preferably mol.wt. of 30,000 to 60,000 daltons). See US 6,302,785.

In another embodiment, the skin engaging member may contain other conventional shaving aid ingredients, such as low mol.wt. water-soluble release enhancing agents such as polyethylene glycol (MW<10,000, e.g., 1-10% by weight PEG-100), water-swellable release enhancing agents such as cross-linked polyacrylics (e.g., 2-7% by weight), colorants, antioxidants, preservatives, vitamin E, aloe, cooling agents, essential oils, beard softeners, astringents, medicinal agents, etc. Portions that contain a colorant can be designed to release the colorant (e.g., by leaching or abrasion), and thereby cause the strip to change color during shaving, preferably in response to wear of the colored portion, so as to provide an indication to the user that the skin engaging member and/or the razor cartridge has reached the end of its effective life or the end of its optimum performance. A portion may contain, for example, between about 0.1% and about 5.0% (preferably between about 0.5% and 3%) colorant by weight.

The matrix can further comprise a water-insoluble polymer in which the water-soluble polymer is dispersed. Preferably, at a level of from about 0% to about 50%, more preferably about 5% to about 40%, and most preferably about 15% to about 35% by weight of the skin engaging member of a water-insoluble polymer. Suitable water-insoluble polymers which can be used include polyethylene (PE), polypropylene, polystyrene (PS), butadiene-styrene copolymer (e.g. medium and high impact polystyrene), polyacetal, acrylonitrile-butadiene-styrene copolymer, ethylene vinyl acetate copolymer, polyurethane, and blends thereof such as polypropylene/polystyrene blend or polystyrene/impact polystyrene blend.

One preferred water-insoluble polymer is polystyrene, preferably a general purpose polystyrene, such as NOVA C2345A, or a high impact polystyrene (i.e. polystyrene-butadiene), such as BASF 495F KG21. The strip or any portion should contain a sufficient quantity of water-insoluble polymer to provide adequate mechanical strength, both during production and use.

### b. Emollients

In another embodiment, the matrix material comprises at least one emollient. In one embodiment the emollient is hydrophobic. In certain embodiments, the composition can consist essentially of one or more emollients which could form a fluid at 25 °C. Where the emollient is fluid form, the fluid is preferably contained within a skin engaging reservoir as disclosed below. In such embodiments, depending on the viscosity of the composition, varying orifice sizes can be used to control the dispensing of emollient during use.

The emollient is liquid, semi-solid and/or solid at room temp. The emollient may comprise one or more hydrocarbon emollients, a lipid, lipophilic skin care actives, or a mixture thereof. Suitable lipids include fatty acyls such as fatty acids, fatty alcohols, esters, triglycerides, fats, butters, and waxes; glycerolipids; glycerophospholipids; sphingolipids; sterol lipids; prenol lipids; saccharolipids; polyketides; lipophilic skin active agent emollients, and mixtures thereof.

Hydrocarbon emollients include straight chain, branched chain, saturated and unsaturated hydrocarbons and mixtures thereof and they may comprise natural or synthetic hydrocarbon emollients and mixtures thereof. Preferred natural hydrocarbon emollients include petrolatum, mineral oil and mixtures thereof. Preferred synthetic hydrocarbon emollients include branched chain hydrocarbons, such as isohexadecane (such as Arlamol HD™ from Croda) and Polydecene (such as Puresyn 2™ from Exxon Mobil).

Fatty alcohol or fatty acid emollients include saturated and unsaturated higher alcohols, especially C₁₂ - C₃₀ fatty alcohols and fatty acids, especially lauric, myristic, palmitic, stearic, arachidic or behenic. Ester emollients include esters of a C₁₂ - C₃₀ alcohol and mixtures thereof, especially isopropyl myristate, isopropyl isostearate and mixtures thereof. Triglyceride emollients include synthetic or natural triglycerides, especially natural triglycerides derived from sunflower, avocado, olive, castor, coconut, cocoa and mixtures thereof. More preferred are coconut-derived triglycerides, such as the commercially available materials Myritol™ 312 and 318 (Cognis), Estasan™ (Croda) and Miglyol™ (Sasol). Fat and butter emollients include coconut butter, shea butter and mixtures thereof. Wax emollients include paraffin wax, microcrystalline wax, candellila , ozokerite and mixtures thereof. Preferably the emollient comprises paraffin wax. Advantageously, hydrophobic phase comprises some wax because waxes may bestow further improved hardness and erodability to the solid moisturising composition. Preferably, the erodible, sold moisturizing composition comprises from 2% to 20% and more preferably from 3% to 15% wax by weight of the erodible, sold moisturizing composition.

Another class of suitable lipids include lipophilic skin active agent emollients which include oil soluble vitamins, such as vitamin E derivatives, including vitamin E acetate and tocopherol nicotinate; oil-soluble vitamin A derivatives, such as retinyl palmitate, lanolin, ceramides, sterols and sterol esters, salicylic acid, camphor, eucalyptol and essential oils.

In one embodiment, the matrix material comprises at least one emollient and a water insoluble structuring polymer. Examples of such compositions have been described as an erodable, solid moisturizing composition described in copending U.S. Patent Application Serial Nos. 61/305682 titled "HAIR REMOVAL DEVICE COMPRISING ERODABLE MOISTURIZER" and 61/305687 titled "HAIR REMOVAL DEVICE COMPRISING AN ERODABLE MOISTURIZER", both to Stephens et al, filed Feb. 18, 2010.

As used herein, the term "solid" when used in relation to the erodable, solid moisturizing composition refers to compositions which are solid at 25°C. As used herein, the term "water-insoluble" when used in relation to the structuring polymer, means "very slightly soluble", according to the United States' Pharmacopeia (USP) definition in 31/NF 26 Vol. 2 General Notices, Page Xvii., or less than "very slightly soluble", which, using the USP definition, means that more than 1000 parts of solvent (water, in this case) are needed to dissolve 1 part of solute (the structuring polymer, in this case) at Standard Temperature and Pressure. As used herein, the term "soluble in" when describing the ability of the water-insoluble structuring polymer to dissolve in the hydrophobic phase means "soluble", according to the United States' Pharmacopeia definition in 31/NF 26 Vol. 2 General Notices, Page Xvii., or less than "soluble", which, using the USP definition, means that less than 30 parts of solvent (the hydrophobic phase, in this case) are needed to dissolve 1 part of solute (the structuring polymer, in this case) at the melting point of the water-insoluble structuring polymer.

In one embodiment, the matrix with the emollient is an erodable, solid moisturizing composition comprised has a Chatillon Hardness at 25°C of about 0.50kg to about 3.25kg, preferably about 0.75 kg to about 3.00kg, more preferably about 1.00kg to about 2.50kg, measured according to the protocol provided hereinbelow. It is believed that a skin conditioning composition having such Chatillon hardness provides beneficial rates of wear. The Chatillon Hardness Test is disclosed in U.S. Patent Application Serial No. 61/305682.

Any water-insoluble structuring polymer comprised within the erodable, solid moisturizing composition may be any water-insoluble structuring polymer which bestows appropriate wear properties to the erodable, solid moisturizing composition and is preferably a water-insoluble structuring polymer which may bestow a Chatillon Hardness in the above-defined ranges to the erodable, solid moisturizing composition. The structuring polymer is water-insoluble to assist miscibility with or solubility in the hydrophobic phase (at the melting point of the water-insoluble structuring polymer), which in turn may ensure a homogenous distribution of hydrophobic phase throughout the polymer and thus more even wear properties. In addition, the water soluble nature of the polymer may improve the durability of the polymer (and therefore also the erodible, solid moisturizing composition) versus more hydrophilic polymers which may solubilise and wash away during hair removal processes that employ water, such as wet shaving.

In one embodiment, the erodable, solid moisturizing composition comprises from 2% to 50%, preferably from 3% to 40%, more preferably 4% to 12% of water-insoluble structuring polymer by weight of the erodable, solid moisturizing composition. In one embodiment, the water-insoluble structuring polymer comprises a block copolymer. More advantageously, the block copolymer comprises a di-block copolymer, a tri-block copolymer, a multi-block copolymer, a radial block copolymer, a random block copolymer, or a mixture of these polymers. More advantageously still, the block copolymer comprises a tri-block copolymer.

In one embodiment, where the matrix material comprise the solid polymeric matrix, one or more emollients can also be included in the solid polymeric matrix.

### c. Carrier

In one embodiment, the skin engaging member further comprises a carrier wherein the matrix material and encapsulated active can be contained within the carrier and/or present on the carrier. As explained above, the matrix material can be a solid polymeric matrix or an emollient in solid or non-solid form. The carrier can be in the form of a tray upon which the matrix material and encapsulated active are applied, or the carrier can form a retaining structure at least partially containing the matrix and encapsulated material. In one embodiment, the carrier forms a reservoir, such as the sheaths disclosed in U.S. Patent No. 6,298,558 and 7,581,318. Where the matrix material comprises an emollient in fluid form, the carrier is preferably a sheath having one or more dispensing orifices to control the dispensing of the emollient. When referring to the compositional make up of the skin engaging member, the weight percentages defined herein are determined based on the components of the skin engaging member disclosed and not the carrier, unless otherwise specified.

### d. Additional Actives in the Matrix

### i. Optional Cooling Agents

The matrix material may also comprise a neat non-volatile cooling agent or an inclusion complex of a skin-soothing agent with a cyclodextrin, preferably in amounts up to about 25%, most preferably 10 to 20%, by weight of the skin engaging member. "Neat" as used herein means that the additional actives are present outside the encapsulates and are dispersed within the remainder of the matrix material. By non-volatile cooling agent is meant an agent which has a physiological cooling effect on the skin and which is appreciably less volatile than menthol. Preferably, the nonvolatile cooling agent will be one which when subjected to thermogravimetric analysis (e.g. using a 951 Thermogravimetric Analyzer from Dupont with a 20°C. temperature rise -^ per minute) will retain at least 50% of its initial weight at a temperature of 160°C., more preferably at least 80% of its initial weight at a temperature of 160°C., and most preferably at least 50% of its initial weight at a temperature of 175°C.

Suitable cooling agents which can be utilized include non-volatile menthol analogs such as menthyl lactate, menthyl ethoxyacetate, menthone glycerinacetal, 3-1menthoxypropane-1,2-diol, ethyl 1-menthyl carbonate, (IS, 3S,4R)-p-menth-8-en-3-ol, menthyl pyrrolidone 25 carboxylate, N-substituted-p-menthane-3-carboxamides (as described in U.S. Pat. No. 4,136, 163) including, for example, N-ethyl-pmenthane-3-carboxamide, acyclic carboxamides

Suitable skin-soothing agents which can be utilized in the cyclodextrin inclusion complex include menthol, camphor, eugenol, eucalyptol, safrol, methyl salicylate, and the aforedescribed menthol analogs. Any suitable cyclodextrin may be utilized to form the inclusion complex including alphacyclodextrin, beta-cyclodextrin, gamma-cyclodextrin and modified cyclodextrins such as hydroxypropyl-beta- cyclodextrin, methyl-beta-cyclodextrin., and acetyl-betacyclodextrin. The preferred cyclodextrins are betacyclodextrin and gamma-cyclodextrin.

When the matrix material comprises a cyclodextrin inclusion complex, the matrix material may also advantageously comprise up to 65 about 10%, preferably about 2 to 7%, by weight of a displacing agent which displaces the skin-soothing agent from the inclusion complex upon contact with water, thereby enhancing the release of the skin-soothing agent from the skin engaging member material during use. The displacing agent is a material which is capable of forming a more stable complex with the cyclodextrin than the complex formed with the skinsoothing agent and, thus, displaces the skin-soothing agent from the complex when the skin engaging member is contacted with water. Suitable displacing agents include surfactants, benzoic acids, and certain amines (e.g. urea). Further details with respect to the aforementioned cooling agents, cyclodextrin inclusion complexes and displacing agents may be found in U.S. Patent Nos. 5,653,971, and, 5,713,131.

Those of skill in the art will understand that one or more of the cooling agents listed in this section as Optional Cooling Agents can also be used as the cooling agent encapsulated within either the nano-particle and/or the micro-particle. The matrix material can further comprise one or more other skin care actives in a neat form. Non-limiting examples of suitable other skin care actives include those disclosed herein in the last paragraph of section IC.

### III. Hair Removal Head

The hair removal device generally comprises a hair removal head and a handle or grip portion, upon which the hair removal head is mounted. The hair removal device can be a manual or power driven and can be used for wet and/or dry application. The hair removal head can include a wide scraping surface such as where the hair removal device is used with a depilatory, or a razor cartridge where the device is a shaving razor. The hair removal head may be replaceable or pivotally connected to a cartridge connecting structure. In an aspect, the cartridge connecting structure includes at least one arm to releasably engage the hair removal head.

The hair removal head comprises one or more elongated edges positioned between said first and said second end, said one or more elongated edges comprising a tip extending towards said first end. Where the hair removal head is a razor cartridge the one or more elongated edges can include blades. For example, U.S. Patent 7,168,173 generally describes a Fusion® razor that is commercially available from The Gillette Company which includes a razor cartridge with multiple blades. Additionally, the razor cartridge may include a guard as well as a shaving aid. A variety of razor cartridges can be used in accordance with the present invention. Nonlimiting examples of suitable razor cartridges, with and without fins, guards, and/or shave aids, include those marketed by The Gillette Company under the Fusion®, Venus® product lines as well as those disclosed in U.S. Patent Nos. 7,197,825, 6,449,849, 6,442,839, 6,301,785, 6,298,558; 6,161,288, and U.S. Patent Publ. 2008/060201. Those of skill in the art will understand that the present skin engaging member can be used with any currently marketed system or disposable razor, including those having 2, 3, 4 or 5 blades. Another example of a hair removal device is a scraping edge for use with a hair removal composition, i.e. a depilatory.

In one embodiment, said at least one skin engaging member is located on the portion of the cartridge that contacts skin during the hair removal process, forward and/or aft of the blades. A feature "forward" of the one or more elongated edges, for example, is positioned so that the surface to be treated with by the hair removal device encounters the feature before it encounters the elongated edges. A feature "aft" of the elongated edge is positioned so that the surface to be treated by the hair removal device encounters the feature after it encounters the elongated edges. Where more than one skin engaging members is provided on the hair removal device, they can be the same or different. By different, meaning having a different carrier, a different skin engaging member, or wherein both sheath and composition are different.

In one embodiment, the cartridge comprises a guard comprising at least one elongated flexible protrusions to engage a user's skin. In one embodiment, at least one flexible protrusions comprises flexible fins generally parallel to said one or more elongated edges. In anther embodiment, said at least one flexible protrusions comprises flexible fins comprises at least one portion which is not generally parallel to said one or more elongated edges. Non-limiting examples of suitable guards include those used in current razor blades and include those disclosed in U.S. Patent Nos. 7,607,230 and 7,024,776; (disclosing elastomeric / flexible fin bars); 2008/0034590 (disclosing curved guard fins); 2009/0049695A1 (disclosing an elastomeric guard having guard forming at least one passage extending between an upper surface and a lower
surface). In one embodiment, said skin engaging member is positioned on the cartridge aft of the guard and forward of said elongated edge. In another embodiment, the skin engaging member is positioned on the cartridge forward of the guard. This embodiment can be particularly useful to deliver the skin engaging member prior to contact with the guard.

### IV. Method of Making

Skin engaging member of the present invention may be fabricated by any appropriate method, including injection molding, pressing, impregnation, spray-coating, calendaring and extrusion, the latter being preferred. All of the components of the strip are blended prior to molding or extrusion. For best results, it is preferred that the components are dry.

The blended components may be extruded through a Haake System 90, ¾ inch diameter extruder with a barrel pressure of about 6.894 x 10⁶ to 13.789 x 10⁶ Pa (1000-2000 psi), a rotor speed of about 10 to 50 rpm, and a temperature of about 150°-185°C and a die temperature of about 170°-185°C. Alternatively, a 1¼ inch single screw extruder may be employed with a processing temperature of 175°-200°C, preferably 185°-190°C, a screw speed of 20 to 50 rpm, preferably 25 to 35 rpm, and an extrusion pressure of 12.410 x 10⁶ to 34.479 x 10⁶ Pa (1800 to 5000 psi), preferably 13.789 x 10⁶ to 24.132 x 10⁶ Pa (2000 to 3500 psi). The extruded strip is air cooled to about 25°C. To injection mold the strips it is preferred to first extrude the powder blend into pellets. This can be done on a 1¼ or 1½ inch single screw extruder at a temperature of 120°-180°C, preferably 140°-150°C, with a screw speed of 20 to 100 rpm, preferably 45 to 70 rpm. The pellets are then molded in either a single material molding or multi-material molding machine, which may be single cavity or multi-cavity, optionally equipped with a hot-runner system. The process temperature can be from 165° to 250°C, preferably from 180° to 225°C. The injection pressure should be sufficient to fill the part completely without flashing. Depending on the cavity size, configuration and quantity, the injection pressure can range from 20.068 x 10⁵ to 17.237 x 10⁶ Pa (300 to 2500 psi). The cycle time is dependent on the same parameters and can range from 3 to 30 seconds, with the optimum generally being about 6 to 15 seconds.

### V. Details on Figures

Referring to Figs. 1 and 2, the razor cartridge 14 includes housing 16, which carries three blades 18, a finned elastomeric guard 20, and a skin engaging member 22 located on a skin-engaging portion (in this case the cap) of the cartridge. Skin engaging member 22 is shown having two layers, the first layer can be the matrix and encapsulated active of the present invention, and the second layer can be a conventional shave aid, or vice versa. The skin engaging member is preferably locked in (via adhesive, a fitment, or melt bonding) an opening in the rear of the uniform, slightly curved to flat upper surface. This type of skin engaging member may also be fabricated in a wedge-shaped cross-section (as shown in Fig 4,. Element 42) or any other desired shape. Skin engaging member may also be constructed in two or more layers, such as a sandwich or a sheath/core construction such as shown in element 52 in Fig.4.

5 Fig. 5 is a thermogravimetric analysis (TGA) of three samples under applied heat at a rate of 10°C/min to 600°C in a nitrogen atmosphere. Sample 1 is an encapsulated active in accordance with the present invention encapsulating L-menthol at a level of 29 % by weight of the encapsulated active. The encapsulated active is sodium starch octenylsuccinate encapsulated L-menthol, available from Salvona. Sample 2 is a DL-menthol contained in a polyurethane capsulate at a level of 30.2 % by total weight. This sample is available from Appleton. The control is pure L-menthol available from Alfa Aesar.

Without intending to be bound by theory, it is believed that the high retention rate of menthol in Sample A is achieved due in part to the specific micro / nano encapsulation. Conventional shave aids are extruded at a temperature range of extrusion process (from about 150 to about180°C). Sample A demonstrates a higher rate of retained material compared to Samples B and the Control. This thermal analysis shows that Sample A can be used in a skin engaging member of the present invention such as a conventional extruded or molded shaving aid.

### VI. Examples

Table 1 provides several exemplary skin engaging members in the form of extruded shaving aids in accordance with the present invention. Each of Samples A - G comprises: from 0.2 to 2 wt % of a nanocapsule wall material comprising wax and/or shea butter; 15 to 30 wt% of a microcapsule wall material comprises starch; 15 to 30 wt% of a shave aid matrix comprising polystyrene (PS), polycaprolactone (PCL), polyvinyl acetate (PVA) or/and ethylene vinyl acetate (Elvax); from 30 to 60 wt % of the shaving aids comprising a polyethylene oxide (PEO) of varying mol.wt. and optionally other shaving aid ingredients such as those disclosed above, the remainder comprising the first active and second active as shown below. The first active and the second actives may be mixed together and present in the nanocapsule, the microcapsule, or both. The first and second actives can also be separated such that one is in the nanocapsule, and the other in the microcapsule.

The L-menthol in Sample A can be in solid particle form. The L-menthol in Sample B is in dissolved in a diluent such as mineral oil and is in liquid form at room temperature. Without intending to be bound by theory, it is believed that the liquid form of cooling agent may be more effective than the solid particle forms when triggered by water or shear and released onto human face during wet shave as compared with solid form. In Sample C weight ratio of L-menthol:Frescolat ML is 1:1, the dual coolants could also form a liquid form which provides long lasting cooling sensation.

**Table 1.**

| SAMPLE | FIRST ACTIVE | SECOND ACTIVE |
|---|---|---|
| A | 5 - 15 wt% L-menthol* | NA |
| B | 5 - 15 wt% L-menthol and mineral oil mixture | NA |
| C | 5 - 10 wt% L-menthol | 0 - 5 wt% Frescolate ML |
| D | 5 - 10 wt% L-menthol | 0 - 5 wt% Frescolate MGA |
| E | 5 - 10 wt% L-menthol | 0-5wt% Coolact 10 |
| F | 5 - 10 wt% L-menthol | 0-5wt% Aloe Vera |
| G | 5 - 10 wt% L-menthol | 0-5wt% Perfume |

| | | |
|---|---|---|
| * L-menthol used in these examples comprise 15-45wt% cooling raw material | | |

**Table 2. Additional exemplary skin engaging elements in the form of extruded shaving aids containing encapsulated L-menthol as cooling agent are described in Samples H to N. All ingredients are provided in wt % of extruded shaving aid. Parameters, such as process temperature, and active retention levels are recorded. The content of L-menthol in the skin engaging element was analyzed via the Gas Chromatographic Method (Agilent 6890N GC) using an internal standard after L-menthol was extracted completely in methanol.**

| | H | I | J | K | L | M |
|---|---|---|---|---|---|---|
| Ingredients | | | | | | |
| Salvona MultiSal Menthol 160^{a} | 33.33 | 45.79 | 32.26 | 16.67 | 32.26 | 32.26 |
| PS^{b} | 13.30 | 16.56 | 19.95 | 16.63 | - | - |
| PEO^{c} | 49.97 | 36.87 | 42.84 | 62.21 | 42.74 | 44.74 |
| PCL^{d} | 3.30 | - | 4.95 | 4.17 | 25.00 | 23.00 |
| Adjunct Ingredients | 0.10 | 0.78 | 0.00 | 0.32 | 0.00 | 0.00 |
| Parameters | | | | | | |
| Extrusion temp (°C) | 160 | 160 | 160 | 160 | 120 | 140 |
| Wt% L-Menthol before extrusion | 9.50 | 13.30 | 9.36 | 5.00 | 9.35 | 9.35 |
| Wt% L-menthol after extrusion | 7.6 | 10.6 | 7.6 | 4.0 | 7.5 | 7.3 |
| % L-menthol retained^{e} | 80.0 | 79.7 | 81.2 | 80.0 | 80.2 | 78.1 |

| | | | | | | |
|---|---|---|---|---|---|---|
| a) From Salvona, 29-30% load of L-menthol b) Polystyrene 731G HIPS (Nova Chemicals) c) Polyox Coag, N750 and Carbowax 4600G (Dow Chemical) d) Polycaprolactone CAPA 6505 (Solvay) e) Value is determined by (L-menthol after extrusion) x 100/(L-menthol before extrusion). | | | | | | |

When Samples H - M were created and tested for % L-menthol retention, each sample gave a retention of greater than 70%. In one embodiment, the skin engaging member of the present invention provides an active in the encapsulated active retention rate of at least about 70%, or at least about 75%, or at least about 80%. Without intending to be bound by theory, it is believed that having a retention rate as described in the previous sentence provides a high enough amount of retained menthol, following the making process, to allow for a user to still feel the benefit of the active.

Comparison Samples N and O are made similar to Sample H but with the encapsulated actives of the present invention replaced with other actives.
Sample N: Encapsulated Salvona MultiSal Menthol 160 is replaced by PUR encapsulated DL-menthol (30.2wt% load) which corresponds to Sample 2 in Fig 5, , the menthol retention has been reduced to 66%. It is believed that a retention rate in this range is insufficiently high to provide the cooling benefit desired.
Sample O: Salvona MultiSal Menthol 160 is replaced by an acrylate encapsulated L-menthol. The L-Menthol retention of Sample O is about 80%. The capsule of the present invention is believed to perform better than the acrylate capsule because acrylate is a non-water soluble material and may act as a waterproofing of the capsule such that the menthol, though retained at a relatively high level is not easily released during the shaving process. The capsules of the present invention, however, are believed to provide more robust release of the actives because the materials used to form the capsules can be disrupted by water and/or shear or pressure.

All parts, ratios, and percentages herein, in the Specification, Examples, and Claims, are by weight and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Except as otherwise noted, the articles "a," "an," and "the" mean "one or more."

It is intended to cover in the appended claims all changes and modifications that are within the scope of this invention.

## Claims

1. A skin engaging member (22) attached to a razor cartridge for use on a hair removal device (14), said skin engaging member comprising:
a. a matrix comprises at least one of: a water soluble polymer, an emollient, and a mixture thereof;
b. an encapsulated active at a level of from 0.01 % to 50% by weight of the skin engaging member, said encapsulate active comprising at least one nano-particle encapsulated in a micro-particle, wherein said nano-particle comprises a shell comprising a hydrophobic material, and wherein said micro-particle comprises a shell comprising a water sensitive material;
wherein at least one of said nano-particle and said micro-particle comprises a skin care active, wherein said microsphere has a size of from 2.0 microns to 100 microns, and wherein said at least one nano-sphere has a size of from 0.01 microns to 5 microns.

2. The skin engaging member of claim 1 or any claim dependant therefrom, wherein said at least one skin care active comprises a cooling agent selected from the group consisting of: L-menthol; p-methane-3,8-diol; Isopulegol; Menthoxypropane-1,2,-diol; Curcumin; Menthyl Lactate; Gingerol; Icilin; Tea Tree Oil; Methyl Salicylate; Camphor; Peppermint Oil; N-Ethyl-p-menthane-3-carboxamide; Ethyl 3-(p-menthane-3-carboxamido)acetate; 2-Isopropyl-N,2,3-trimethylbutyramide; Menthone glycerol ketal, Menthone Glyerine Acetal; and mixtures thereof.

3. The skin engaging member of claim 2 or any claim dependant therefrom, wherein said cooling agent is a mixture of menthol and menthyl lactate in a ratio of weight in the range of 1:4 to 4:1

4. The skin engaging member of any preceding claim, wherein said hydrophobic material is selected from the group consisting of alkylated polyvinyl pyrolidine, hydrogenated castor oil, hydrogenated vegetable oil, hard paraffin, hard fat, triglyceride, and mixtures thereof.

5. The skin engaging member of any preceding claim, wherein said water sensitive material comprises one or more of: a water soluble and water dispersible synthetic polymers and copolymer, a starch derivative, a polysaccharide, a hydrocolloid, a natural gum, a protein, and a mixture thereof.

6. The skin engaging member of claim 1 or any claim dependant therefrom, wherein the water sensitive material comprises polyvinyl alcohol having a degree of hydrolysis of from 75% to 100%.

7. The skin engaging member of any preceding claim, wherein said hydrophobic material is selected from the group consisting of alkylated polyvinyl pyrolidine, hydrogenated castor oil, hydrogenated vegetable oil, hard paraffin, hard fat, triglyceride, and mixtures thereof; wherein said water sensitive material comprises one or more of: a water soluble and water dispersible synthetic polymers and copolymer, a starch derivative, a polysaccharide, a hydrocolloid, a natural gum, a protein, and a mixture thereof

8. The skin engaging member of any preceding claim, wherein said water soluble polymer of the matrix comprises at least one of a polyethylene oxide, polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and a mixtures thereof.

9. The skin engaging member of claim 8 or any claim dependant therefrom, wherein said level of water soluble polymer is at a level of from about 50% to about 99% by weight of said solid polymeric matrix.

10. The skin engaging member of any preceding claim wherein said matrix further comprises a water insoluble polymer comprises at least one of: polyethylene, polypropylene, polystyrene, high impact polystyrene, butadiene styrene copolymer, polyacetal, acrylonitrile-butadiene styrene copolymer, ethylene vinyl acetate copolymer, and mixtures thereof.

11. The skin engaging member of claim 10, wherein said water insoluble polymer is present at a level of at least 35% by weight of said solid polymeric matrix.

12. The skin engaging member of any preceding claim, wherein said matrix material comprises an emollient.

13. The skin engaging member of claim 12, wherein said matrix further comprises a block polymer selected from the group consisting of: a di-block copolymer, a tri-block copolymer, a multi-block copolymer, a radial block copolymer, a random block copolymer, and mixtures thereof.

## Patentansprüche

1. Hauteingriffselement (22), das an einer Rasierklingeneinheit befestigt ist, zur Verwendung an einer Haarentfernungsvorrichtung (14), wobei das Hauteingriffselement Folgendes umfasst:
a. eine Matrix, die wenigstens eines von Folgendem umfasst: ein wasserlösliches Polymer, einen Weichmacher, und einer Mischung davon;
b. einen verkapselten Wirkstoff zu einem Gehalt von 0,01 Gew.-% bis 50 Gew.-% des Hauteingriffselementes, wobei der verkapselte Wirkstoff wenigstens ein Nanopartikel umfasst, das in einem Mikropartikel verkapselt ist, wobei das Nanopartikel eine Hülle umfasst, die ein hydrophobes Material umfasst, und wobei das Mikropartikel eine Hülle umfasst, die ein wasserempfindliches Material umfasst;
wobei wenigstens eines von dem Nanopartikel und dem Mikropartikel einen Hautpflegewirkstoff umfasst, wobei die Mikrokugel eine Größe von 2,0 Mikrometer bis 100 Mikrometer aufweist, und wobei die wenigstens eine Mikrokugel eine Größe von 0,01 Mikrometer bis 5 Mikrometer aufweist.

2. Hauteingriffselement nach Anspruch 1 oder einem davon abhängigen Anspruch, wobei der wenigstens eine Hautpflegewirkstoff ein Kühlmittel umfasst, das ausgewählt ist aus der Gruppe bestehend aus: L-Menthol; p-Methan-3,8-Diol; Isopulegol; Menthoxypropan-1,2,-Diol; Curcumin; Menthyllactat; Gingerol; Icilin; Teebaumöl; Methylsalicylat; Kampfer; Pfefferminzöl; N-Ethyl-p-menthan-3-carboxamid; Ethyl 3-(p-Menthan-3-carboxamido)acetat; 2-Isopropyl-N,2,3-trimethylbutyramid; Menthonglycerolketal, Menthonglyerinacetal; und Mischungen davon.

3. Hauteingriffselement nach Anspruch 2 oder jedem beliebigen, davon abhängigen Anspruch, wobei das Kühlmittel eine Mischung aus Methanol und Menthyllactat in einem Gewichtsverhältnis im Bereich von 1:4 bis 4:1 ist.

4. Hauteingriffselement nach einem der vorstehenden Ansprüche, wobei das hydrophobe Material ausgewählt ist aus der Gruppe bestehend aus alkyliertem Polyvinylpyrolidin, hydriertem Castoröl, hydriertem Pflanzenöl, Hartparaffin, Hartfett, Triglycerid, und Mischungen davon.

5. Hauteingriffselement nach einem der vorstehenden Ansprüche, wobei das wasserempfindliche Material eines oder mehrere umfasst aus: einem wasserlöslichen und wasserdispergierbaren, synthetischen Polymer und Copolymer, einem Stärkederivat, einem Polysaccharid, einem Hydrokolloid, einem Naturgummi, einem Protein, und einer Mischung davon.

6. Hauteingriffselement nach Anspruch 1 oder ein beliebiger, davon abhängiger Anspruch, wobei das wasserempfindliche Material Polyvinylalkohol mit einem Hydrolysegrad von 75 % bis 100 % aufweist.

7. Hauteingriffselement nach einem der vorstehenden Ansprüche, wobei das hydrophobe Material ausgewählt ist aus der Gruppe bestehend aus alkyliertem Poly-vinylpyrolidin, hydriertem Castoröl, hydriertem Pflanzenöl, Hartparaffin, Hartfett, Triglycerid, und Mischungen davon; wobei das wasserempfindliche Material eines oder mehrere umfasst aus: einem wasserlöslichen und wasserdispergierbaren synthetischen Polymer und Copolymer, einem Stärkederivat, einem Polysaccharid, einem Hydrokolloid, einem Naturgummi, einem Protein, und einer Mischung davon.

8. Hauteingriffselement nach einem der vorstehenden Ansprüche, wobei das wasserlösliche Polymer der Matrix wenigstens eines aus einem Polyethylenoxid, Polyvinylpyrrolidon, Polyacrylamid, Polyhydroxymethacrylat, Polyvinylimidazolin, Polyethylenglycol, Polyvinylalkohol, Polyhydroxyethymethacrylat, Siliconpolymere, und Mischungen davon umfasst.

9. Hauteingriffselement nach Anspruch 8 oder ein beliebiger, davon abhängiger Anspruch, wobei der Gehalt an wasserlöslichem Polymer bei einem Gehalt von etwa 50 Gew.-% bis etwa 99 Gew.-% der Festpolymermatrix liegt.

10. Hauteingriffselement nach einem der vorstehenden Ansprüche, wobei die Matrix ferner ein wasserunlösliches Polymer umfasst, das wenigstens eines von Folgendem umfasst: Polyethylen, Polypropylen, Polystyrol, hochschlagfestes Polystyrol, Butadien-Styrolcopolymer, Polyacetal, Acrylonitril-Butadien-Styrolcopolymer, Ethylenvinyl-Acetatcopolymer, und Mischungen davon.

11. Hauteingriffselement nach Anspruch 10, wobei das wasserunlösliche Polymer zu einem Gehalt von wenigstens 35 Gew.% der Festpolymermatrix vorliegt.

12. Hauteingriffselement nach einem der vorstehenden Ansprüche, wobei das Matrixmaterial einen Weichmacher umfasst.

13. Hauteingriffselement nach Anspruch 12, wobei die Matrix ferner ein Blockpolymer umfasst, ausgewählt aus der Gruppe bestehend aus: einem di-Blockcopolymer, einem tri-Blockcopolymer, einem multi-Blockcopolymer, einem Radialblockcopolymer, ein Random-Blockcopolymer, und Mischungen davon.

## Revendications

1. Élément de mise en prise de peau (22) fixé à une cartouche de rasoir destinée à être utilisée sur un dispositif d'élimination de poils (14), ledit élément de mise en prise de peau comprenant :
a. une matrice comprenant au moins l'un parmi : un polymère hydrosoluble, un émollient, et un mélange de ceux-ci ;
b. un agent actif encapsulé à un taux allant de 0,01 % à 50 % en poids de l'élément de mise en prise de peau, ledit agent actif encapsulé comprenant au moins une nanoparticule encapsulée dans une microparticule, dans lequel ladite nanoparticule comprend une enveloppe comprenant un matériau hydrophobe, et dans lequel ladite microparticule comprend une enveloppe comprenant un matériau sensible à l'eau ;
dans lequel au moins l'une parmi ladite nanoparticule et ladite microparticule comprend un principe actif pour le soin de la peau, dans lequel ladite microsphère a une taille allant de 2,0 micromètres à 100 micromètres, et dans lequel ladite au moins une nanosphère a une taille allant de 0,01 micromètre à 5 micromètres.

2. Élément de mise en prise de peau selon la revendication 1 ou l'une quelconque revendication dépendant de celle-ci, dans lequel ledit au moins un principe actif pour le soin de la peau comprend un agent de refroidissement choisi dans le groupe constitué de : L-menthol ; p-méthane-3,8-diol ; isopulégol ; menthoxypropane-1,2,-diol ; curcumine ; lactate de menthyle ; gingérol ; iciline ; huile de théier ; salicylate de méthyle ; camphre ; essence de menthe poivrée ; N-éthyl-p-menthane-3-carboxamide ; 3-(p-menthane-3-carboxamido)acétate d'éthyle ; 2-isopropyl-N,2,3-triméthylbutyramide ; menthone glycérol cétal, menthone glycérine acétal ; et des mélanges de ceux-ci

3. Élément de mise en prise de peau selon la revendication 2 ou l'une quelconque revendication dépendant de celle-ci, dans lequel ledit agent de refroidissement est un mélange de menthol et de lactate de menthyle dans un rapport de poids dans la plage de 1:4 à 4:1

4. Élément de mise en prise de peau selon l'une quelconque des revendications précédentes, dans lequel ledit matériau hydrophobe est choisi dans le groupe constitué de polyvinyl-pyrolidine alkylée, huile de ricin hydrogénée, huile végétale hydrogénée, paraffine dure, matière grasse dure, triglycéride, et des mélanges de ceux-ci.

5. Élément de mise en prise de peau selon l'une quelconque des revendications précédentes, dans lequel ledit matériau sensible à l'eau comprend un ou plusieurs parmi : des polymères et copolymères synthétiques hydrosolubles et hydrodispersibles, un dérivé d'amidon, un polysaccharide, un hydrocolloïde, une gomme naturelle, une protéine, et un mélange de ceux-ci.

6. Élément de mise en prise de peau selon la revendication 1 ou l'une quelconque revendication dépendant de celle-ci, dans lequel le matériau sensible à l'eau comprend un alcool polyvinylique possédant un degré d'hydrolyse allant de 75 % à 100 %.

7. Élément de mise en prise de peau selon l'une quelconque des revendications précédentes, dans lequel ledit matériau hydrophobe est choisi dans le groupe constitué de polyvinyl-pyrolidine alkylée, huile de ricin hydrogénée, huile végétale hydrogénée, paraffine dure, matière grasse dure, triglycéride, et des mélanges de ceux-ci ; dans lequel ledit matériau sensible à l'eau comprend un ou plusieurs parmi : des polymères et copolymères synthétiques hydrosolubles et hydrodispersibles, un dérivé d'amidon, un polysaccharide, un hydrocolloïde, une gomme naturelle, une protéine, et un mélange de ceux-ci.

8. Élément de mise en prise de peau selon l'une quelconque des revendications précédentes, dans lequel ledit polymère hydrosoluble de la matrice comprend au moins l'un parmi un oxyde de polyéthylène, une polyvinylpyrrolidone, un polyacrylamide, un polyhydroxyméthacrylate, une polyvinylimidazoline, du polyéthylène glycol, un alcool polyvinylique, un polyhydroxyéthylméthacrylate, des polymères de silicone, et des mélanges de ceux-ci.

9. Élément de mise en prise de peau selon la revendication 8 ou l'une quelconque revendication dépendant de celle-ci, dans lequel ledit taux de polymère hydrosoluble est à un taux allant d'environ 50 % à environ 99 % en poids de ladite matrice polymère solide.

10. Élément de mise en prise de peau selon l'une quelconque des revendications précédentes, dans lequel ladite matrice comprend en outre un polymère insoluble dans l'eau comprenant au moins l'un parmi : du polyéthylène, du polypropylène, du polystyrène, du polystyrène à haut impact, un copolymère butadiène styrène, du polyacétal, un copolymère acrylonitrile-butadiène styrène, un copolymère éthylène-acétate de vinyle, et des mélanges de ceux-ci.

11. Élément de mise en prise de peau selon la revendication 10, dans lequel ledit polymère insoluble dans l'eau est présent à un taux d'au moins 35 % en poids de ladite matrice polymère solide.

12. Élément de mise en prise de peau selon l'une quelconque des revendications précédentes, dans lequel ledit matériau de matrice comprend un émollient.

13. Élément de mise en prise de peau selon la revendication 12, dans lequel ladite matrice comprend en outre un polymère séquencé choisi dans le groupe constitué de : un copolymère di-séquencé, un copolymère tri-séquencé, un copolymère multi-séquencé, un copolymère séquencé radial, un copolymère séquencé statistique, et des mélanges de ceux-ci.
